# EUROPEAN PATENT APPLICATION

(11) **EP 2 253 336 A1**
(43) Date of publication of application: **24.11.2010**
(21) Application number: 10162492.2
(22) Date of filing: 11.05.2010
(51) Int. Cl.: A61L 15/40, A61L 15/46

(54) **A silk medical device with antimicrobial properties and a method of manufacture thereof**

(30) Priority: 15.05.2009 US 178862 P
(71) Applicant: Spintec Engineering GmbH, 52066 Aachen (DE)
(72) Inventor: Rheinnecker, Michael, 52066, Aachen (DE); Zimmat, Rolf, 52066, Aachen (DE); Willmann, Charlotte, 52066, Aachen (DE)
(74) Representative: Harrison, Robert John

(57) **Abstract**

A silk medical device (60). The silk medical device (60) is manufactured from a silk protein material (15) which is loaded with a polymeric cationic antimicrobial (55). The polymeric cationic antimicrobial (55) is polyhexamethylene biguanide (PHMB). The silk medical device (60) is used for the treatment of wounds (70) to prevent infection.

## Description

### Description

### Title of the Invention

A silk medical device with antimicrobial properties and a method of manufacture thereof.

### Cross Relation to Other Applications

The present application claims benefit and priority of US provisional patent application No. 61/178,862 filed on 15 May 2009.

### Field of Invention

The field of the present invention relates to medical devices. The medical device comprises a silk material which is loaded with a polymeric cationic antimicrobial compound.

### Background of Invention

A silk material is a material such as natural or man-made silk material. The silk material comprises proteins and peptides and the silk material is derived from a silk producing organism, such as for example silkworms, spiders and mussels.

Due to the advent of modern biotechnological methods, the silk material can be synthetically manufactured. The synthetic manufacture of the silk material allows the manufacture of the silk material with many controllable and different material and mechanical properties (see for example, Altman et al., Biomaterials 2003, 24: 401-416).

International patent application No. PCT/US2005/020844 by Kaplan et al. is titled "Silk based drug delivery system". The Kaplan et al. document discloses a method for the manufacture of a pharmaceutical formulation for the controlled release of a therapeutic agent. The method proceeds by contacting a silk fibroin solution with the therapeutic agent to form a silk fibroin article comprising the therapeutic agent. The crystalline conformation of the silk fibroin article is altered to control the release of the therapeutic agent from the silk fibroin article. The crystalline conformation of the silk fibroin article is altered, for example, using an alcohol, pressure, an electric field, salts or a shear force in order to control the diameter of pores within the silk fibroin article, thus enabling a controlled release of the therapeutic agent from the silk fibroin article.

The altering of the crystalline conformation of the silk fibroin article using an alcohol, pressure, the electric field, salts or the shear force can reduce or destroy the activity of the therapeutic agent within the pharmaceutical formulation.

US patent application NO. 12/025524 by Arai et al. is titled "Biodegradable biopolymers, methods for their preparation and functional materials constituted by these biopolymers". The Arai et al. document discloses the preparation of a biodegradable biopolymer by applying onto a substrate an aqueous solution of silk fibroin solution and a secondary substance such as cellulose, chitin or keratin. The biodegradable biopolymer is immersed in an aqueous solution containing antibacterial metal ions such as silver, copper and/or cobalt. The biodegradable biopolymer containing the antibacterial metal ions is used as an antibacterial device by the action of an enzyme which decomposes the biodegradable biopolymer.

A further disclosure by Arai et al. (Journal of Applied Polymer Science 2001, 80: 297-303) demonstrates that silk can absorb and bind cations (i.e. positively charged metal ions). However, as demonstrated by the Journal of Applied Polymer Science 2001, 80: 297-303 disclosure and the US patent application NO. 12/025524 disclosure, the exact nature of the interaction of the cations with silk, particularly their absorption and release kinetics, can vary between different cations. The absorption and release kinetics of the cations from the silk depends on factors such as pH, temperature and the presence of additives.

There is a need to provide a silk medical device that can be used in medical applications that provides a pharmacologically active substance such as an antimicrobial compound which is released rapidly from the silk medical device and at high levels to provide rapid onset of pharmacological action. Given the variability known for metal ion binding to the silk protein and the release of the metal ions from the silk protein, it is impossible for a person skilled in the art to predict the strength and nature of an ionic or a hydrogen bonding of larger, polymeric cationic substances to the silk proteins. Depending on the particular cationic substance used, the ionic or hydrogen bonding may be strong due to a large number of electrostatic interactions or possible hydrogen bonds or weak due to the inaccessibility of negatively charged surface areas or hydrogen-bond donating groups on the silk proteins.

The release kinetics of cationic antimicrobial compounds from non-protein and non-peptide materials (for example cellulose or man-made polymers) is known in the art. Polymeric cationic antimicrobial compounds are numerous and can be synthetically manufactured. An example of a poly cationic antimicrobial compound is polyhexamethylene biguanide (PHMB).

Technologies are known in the art which facilitate the release of PHMB with fast or slow release kinetics from non-protein and non-peptide materials (i.e. not silk materials). International patent application No. PCT/EP2005/013340 by Fugmann and Dietze is titled "Infection resistant polyurethane foams, method for producing the same and use thereof in antiseptic wound dressings". The Fugmann and Dietze document discloses a microbiocidal polyurethane foam comprising PHMB and a superabsorbent material.

European patent No. EP 1473047 by Xylos Corp. is titled "Microbial cellulose wound dressing sheet, containing PHMB for treating chronic wounds". The Xylos Corp patent discloses a manufacturing method which provides a wound dressing sheet which allows the fast release of PHMB from the microbial cellulose wound dressing sheet. The Xylos Corp patent fails to disclose a silk medical device loaded with PHMB. The Xylos Corp patent also fails to disclose a method for the manufacture of a silk medical device loaded with PHMB which releases PHMB at therapeutically relevant levels and with known release profiles.

US patent application No. US 10/278,072 by Harish Patel is titled, "Medical dressing containing antimicrobial agent". The Harish Patel patent application discloses the manufacture of a layered cellulose-polyester wound dressing which facilitates a slow release of PHMB from the wound dressing. The Harish Patel patent application fails to disclose a silk medical device loaded with PHMB. The Harish Patel patent application also fails to disclose a method for the manufacture of a silk medical device loaded with PHMB which releases PHMB at therapeutically relevant levels and with known release profiles.

International patent application NO. PCT/GB2004/004738 by Arch UK Biocides Ltd, is titled "Fibres treated with antimicrobial agents". The Arch UK Biocides Ltd document discloses the use of a self crosslink able resin and a catalyst to permanently immobilise PHMB to non-cellulosic fibres in order to prevent a release of PHMB from the non-cellulosic material and ensure durability to laundering or rinsing. The Arch UK Biocides Ltd document fails to disclose a silk medical device loaded with PHMB. The Arch UK Biocides Ltd document also fails to disclose a method for the manufacture of a silk medical device loaded with PHMB which releases PHMB at therapeutically relevant levels and with known release profiles.

When evaluating PHMB loaded cellulose or PBMH loaded non-protein and non-peptide based materials disclosed by the prior art, it should be noted that the silk material has a different molecular structure and composition. Cellulose is known to exhibit a highly uniform molecular structure which is made up of one simple, low molecular weight carbohydrate (glucose) to which PHMB can bind through electrostatic and hydrogen-bonding interactions as described by Blackburn et al. (see for example Langmuir 2006, 22: 5636-5644). In contrast to cellulose, the silk materials comprise very large (2.3 MDa) multi-domain protein complexes (elementary units), which comprise six sets of a disulfide-linked heavy chain/light chain fibroin hetero-dimer and one molecule of P25 (see for example Inoue et al in JBC 2000, 275, 40517-40528). Given the enormous size and complexity of those elementary units of the silk material, it is - unlike for cellulose - not possible to predict the strength of electrostatic or hydrogen-bonding interactions between PHMB and the elementary units of the silk material.

There is no prior art known which teaches a method for the manufacture of a silk medical device comprising a polymeric cationic antimicrobial material, which allows for the rapid or slow release of the polymeric cationic antimicrobial material from the silk medical device. There is no prior art that teaches a silk medical device comprising polymeric cationic antimicrobial material, which allows for the rapid or slow release of the polymeric cationic antimicrobial material from the silk medical device.

### Summary of Invention

An object of the present disclosure is to provide an improved medical device made from a silk protein membrane, fibres and combinations thereof.

The disclosure teaches a method for the manufacture of a medical device having the following steps: providing a silk protein material, impregnating the silk protein material with a polymeric cationic antimicrobial material, and drying the impregnated silk protein membrane.

The polymeric cationic antimicrobial is in one aspect of the invention, polyhexamethylene biguanide (PHMB).

The disclosure also teaches a medial device made from a silk protein material impregnated with the polymeric cationic antimicrobial. The silk medical device releases polymeric cationic antimicrobials at the site of application or implantation. The silk medical device enables the prevention of an infection or allows treatment of an already infected wound.

### Brief Description of Figures

Figure 1 shows the PAGE analysis of the silk material used.
Figure 2 shows the dry and wet weights of the silk material before and after loading with PHMB.
Figure 3 shows the PHMB release from the silk medical device according to the present invention.
Figure 4 shows the PHMB release from Suprasorb X + PHMB wound dressings.
Figure 5 shows the calculated and measured amounts of PHMB uptake by a silk material according to the present invention.
Figure 6 shows the PHMB uptake of a silk medical device at different pH values according to the present invention.
Figure 7 shows the PHMB release from a silk medical device during different lengths of time according to the present invention.
Figure 8 shows the PHMB uptake by silk textile and membrane samples.
Figure 9 shows the PHMB release from silk fibres loaded with PHMB according to the present invention.
Figure 10 shows a scheme for a method of manufacture of a silk medical device loaded with PHMB according to the present invention.

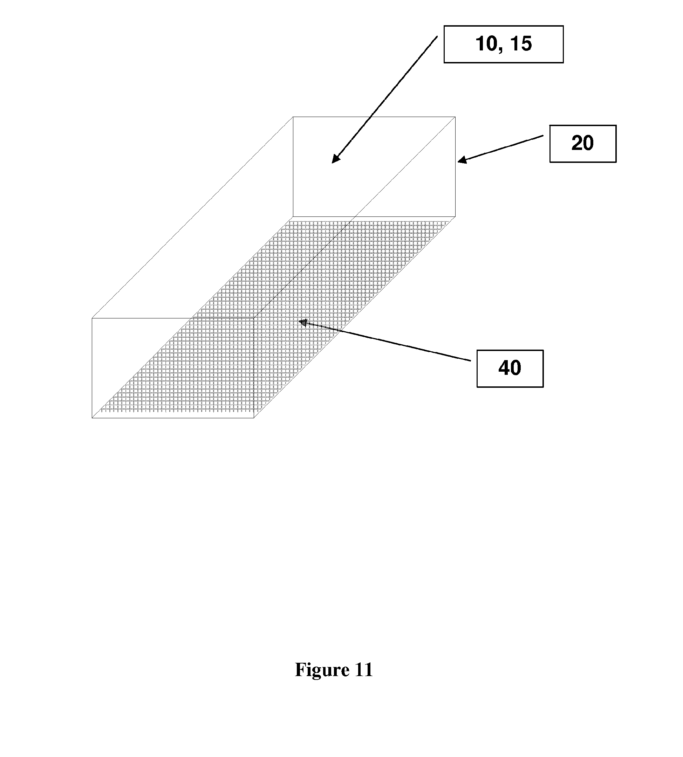

Figure 11 shows an apparatus for the manufacture of a silk medical device loaded with PHMB according to the present invention.
Figure 12 shows a silk medical device according to the invention.
Figure 13 shows the PHMB uptake for silk protein membranes with different thicknesses.

### Detailed Description of the Invention

For a complete understanding of the present invention and the advantages thereof, reference is made to the following detailed description taken in conjunction with the accompanying figures.

It should be appreciated that the various aspects and embodiments of the present invention disclosed herein are merely illustrative of specific ways to make and use the invention and do not therefore limit the scope of invention when taken into consideration with the appended claims and the following detailed description and the accompanying figures.

It should be realised that features from one aspect and embodiment of the invention will be apparent to those skilled in the art from a consideration of the specification or practice of the invention disclosed herein and these features can be combined with features from other aspects and embodiments of the invention.

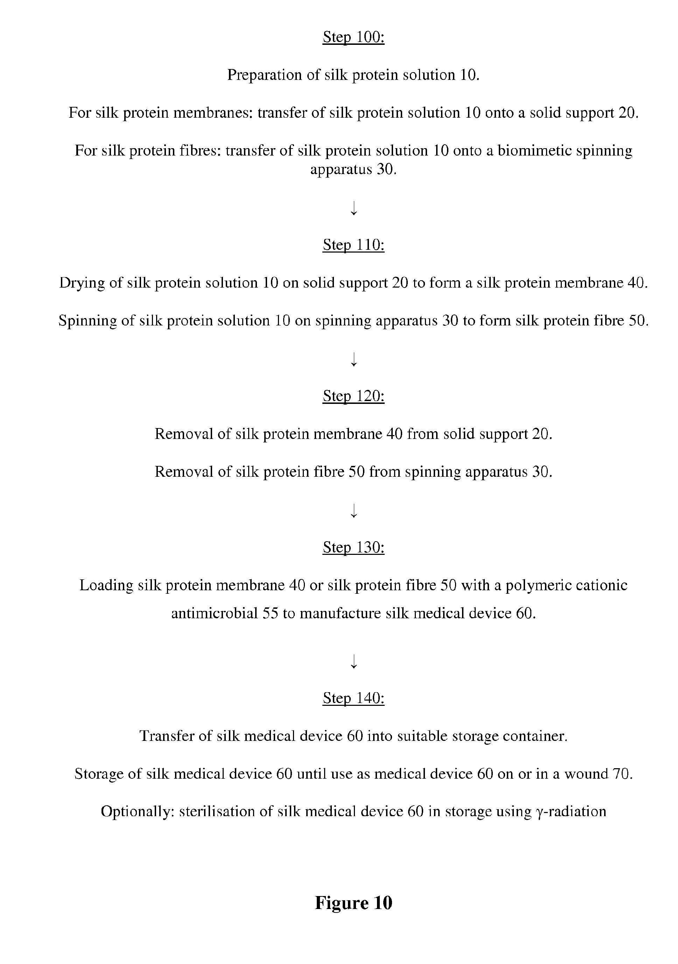

Figure 10 shows a scheme for a method of manufacture of a silk medical device 60 loaded with a poly cationic antimicrobial compound for example polyhexamethylene biguanide (PHMB) according to the present invention.

In a first step 100, a silk protein solution 10 is prepared. The silk protein solution 10 has a silk protein content of between 0.3 and 30% (w/w). The silk protein solution 10 is prepared using a water-based solvent, for example but not limited to deionised water. The silk protein solution 10 is prepared for example as described in US patent No. 7041797.

The silk protein solution 10 is then used for the manufacture of a silk protein material 15. The silk protein material 15 can be made in the form of a silk protein membrane 40 or in the form of a silk protein fibre 50.

The silk protein membrane 40 is formed by transferring the silk protein solution 10 onto a solid support 20. The solid support 20 can be made out of glass or polytetrafluoroethylene (PTFE) or other materials suitable for use with the silk proteins.

The silk protein fibre 50 is formed by transferring the silk protein solution 10 to a biomimetic spinning apparatus 30, where the silk protein fibre 50 is formed by spinning. The silk protein fibre 50 is formed as described by the present Applicants in European Patent No. EP 1244828 and international patent application No. WO 2008/052755.

In step 110, the silk protein solution 10 is dried on the solid support 20 to form the silk protein material 15 which forms the silk protein membrane 40. The length of time to dry the silk protein solution 10 depends on the protein content of the silk protein solution 10 and the rate of evaporation of the solvent from the silk protein solution 10. For drying at room temperature and normal pressure, the drying time of the silk protein solution 10 can vary from between 8 hours and 48 hours, when the silk protein solution 10 has a 1 - 10% silk protein content. The rate of evaporation may be varied for example through the use of vacuum techniques.

Alternatively, the silk protein solution 10 is spun by the spinning apparatus 30 to form the silk protein fibre 50.

In the next step 120, the formed silk protein material 15, be it either the silk protein membrane 40 or the silk protein fibre 50 are removed from the solid support 20 or the spinning apparatus 30, respectively.

In step 130, the formed silk protein material 15 is loaded with a polymeric cationic antimicrobial 55 to manufacture a silk medical device 60. The silk protein material 15 is loaded with the polymeric cationic antimicrobial 55 through impregnation techniques. One example of the polymeric cationic antimicrobial 55 is polyhexamethylene biguanide (PHMB). The exact conditions for the impregnation of the polymeric cationic antimicrobial 55 with the silk protein material 15 depend on a concentration of a solution of the polymeric cationic antimicrobial 55, temperature and a thickness of the silk protein material 15 used.

It was found that the incubation of a 60 µm thick silk protein membrane 40 in a 5% PHMB solution over night and at room temperature was sufficient to manufacture the silk medical device 60 which allow release of PHMB with similar amounts and release kinetics as a commercially available PHMB loaded cellulose dressing such as for example Suprasorb X + PHMB (Lohmann Rauscher).

In the next step 140, the silk protein membrane 40 or the silk protein fibre 50 loaded with the polymeric cationic antimicrobial 55 is transferred into a suitable container and stored until further use as the silk medical device 60. The silk medical device 60 can be used on a wound 70 on skin 80.

The manufactured silk medical device 60, can be sterilised inside a storage container by the use of γ-radiation.

### Examples

The following examples of specific embodiments for carrying out the present invention are offered for illustrative purposes only, and are not intended to limit the scope of the present invention in any way.

### Example 1

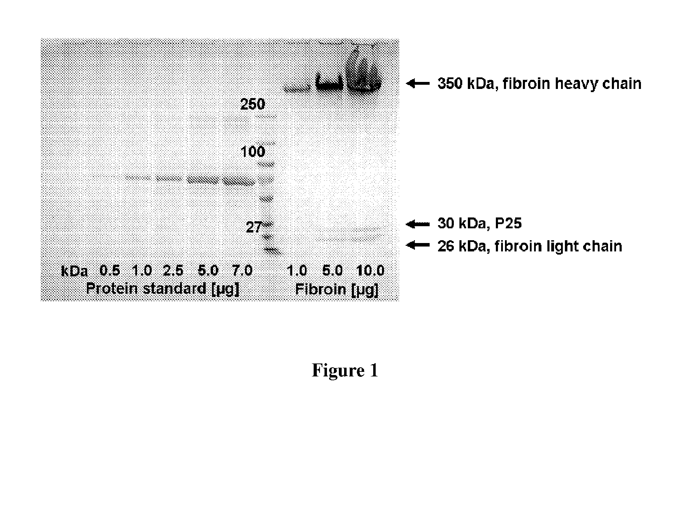

The silk protein solution 10 was prepared according to the method described by the present applicant in WO 2007/098951. The purity of the silk protein, present in the silk protein solution 10 was assessed by PAGE (see figure 1). The PAGE analysis confirms that the purity of the silk protein used for manufacture of the silk membranes 15 is greater than 95%.

### Example 2

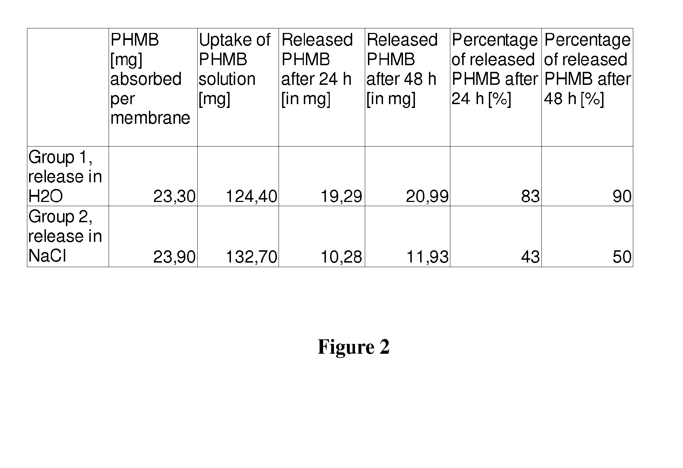

Silk protein membranes 40 (50 x 50 x 0.06 mm) were prepared according to the method described by the Applicant in international patent publication No. WO 2007/098951. 16 holes each of diameter 5 mm each were punched into each silk protein membranes 40. The silk protein membranes 40 were washed over-night in distilled water. The weight of each individual one of the silk protein membranes 40 was recorded (dry weight 1). Each silk protein membranes 40 was then immersed separately in 20 ml of a 5% PHMB aqueous solution on a shaker for 16 hours at 60 rpm. As a negative control, the silk protein membranes 40 were incubated in 20 ml deionised H₂O for 16 hours at 60 rpm. Each of the silk protein membranes 40 was then removed from the PHMB solution and weighted (wet weight 1). The silk protein membranes 40 loaded with the PHMB was allowed to dry overnight at room temperature. The weight of each resultant dried silk medical device 60 was recorded (dry weight 2). The amount of PHMB (mg) absorbed by the silk protein membranes 40 was calculated as the difference between dry weight 2 and dry weight 1 (see figure 2 - PHMB absorbed per membrane). The amount of the PHMB loading solution taken up by each one of the silk protein membranes 40 was calculated by the difference between wet weight 1 and dry weight 1 (see figure 2 - uptake of PHMB solution).

The release profile of the polymeric cationic antimicrobial 55 (PMBH) from the manufactured silk medical device 60 was studied in water and in the presence of salts. A commercially available PHMB loaded wound dressings (Suprasorb X + PHMB, Lohmann & Rauscher, 5 x 5 cm) was used as controls in the assay. The amount of PHMB released and the release kinetics of the Suprasorb samples were used as internal standard to define the target PHMB release profile of a therapeutically relevant wound dressing for infected wounds.

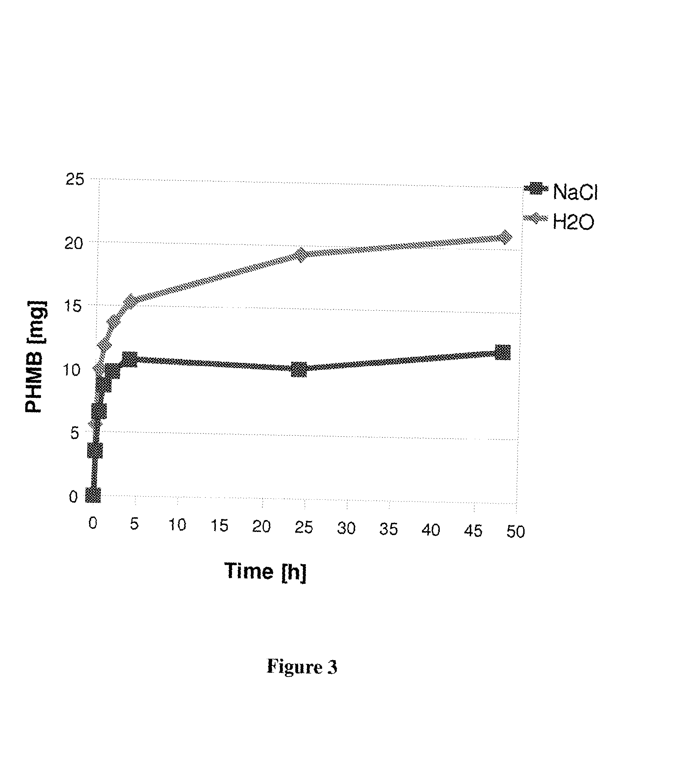

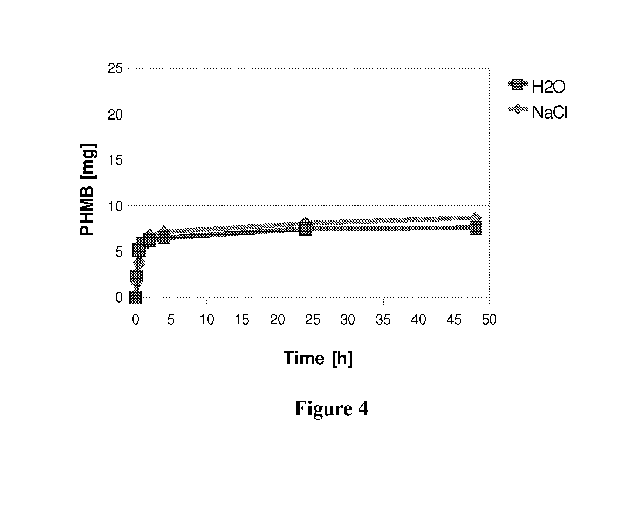

The silk medical device 60 and the Suprasorb samples were split into two groups and incubated individually in either 20 ml d H₂0 (group 1) or 20 ml 0.9 % NaCl (group 2) at 37 °C in petri dishes at 60 rpm. The release of the polymeric cationic antimicrobial 55 (PMBH) from each sample was then measured by taking samples at 0, 10, 30 minutes, 1, 2, 4, 24 and 48 hour intervals. The polymeric cationic antimicrobial 55 (PMBH) concentration was determined by spectroscopic analysis at 236 nm and calculated using a freshly prepared calibration curve. The total polymeric cationic antimicrobial 55 (PMBH) released over time in dH₂O (group 1) and in 0.9 % NaCl (group 2) are displayed in figure 3 (silk medical device 60) and figure 4 (Suprasorb). The results of figure 3 and figure 4 demonstrate that the silk medical device 60 shows a faster onset of polymeric cationic antimicrobial 55 (PMBH) release with 15.3 mg released in d H₂O and 10.8 mg released in 0.9 % NaCl after 4 hours, compared to only 6.5 mg in d H₂O and 7.1 mg in 0.9 % NaCl after 4 hours for the Suprasorb samples. After 48 hours, silk medical device 60 released 20.1 mg PHMB in dH₂O and 11.9 mg in NaCl, compared with 7.6 mg in d H₂O and 8.6 mg in NaCl for Suprasorb. The results confirm that the silk protein membranes 40 can be manufactured to comprise a PHMB release profile which compares well with that of a commercially available PHMB wound dressing. The negative control samples (membranes without PHMB) did not show any release of PHMB (data not shown).

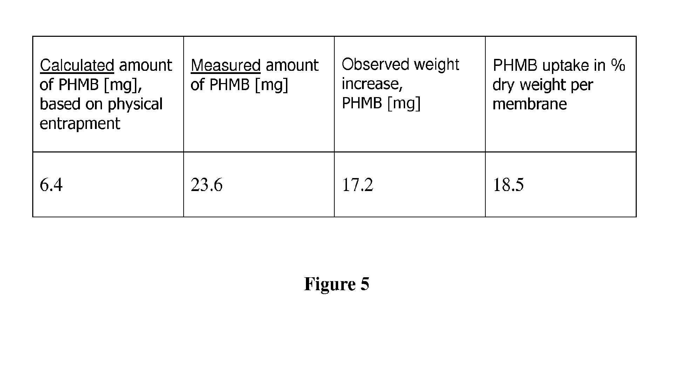

Assuming physical entrapment of the polymeric cationic antimicrobial 55 (PMBH) in the silk protein membrane 40 during evaporation of the solvent without any specific adsorption of the polymeric cationic antimicrobial 55 (PMBH) to the silk protein membrane 40, the weight increase should be equivalent to the amount of the polymeric cationic antimicrobial 55 (PMBH) in the polymeric cationic antimicrobial 55 (PMBH) loading solution which is taken up by each membrane (see figure 2 - uptake of PHMB solution). Therefore, the predicted weight increase (see figure 5 - calculated amount of PHMB) after loading with PHMB should have been 6.22 mg PHMB (group 1) and 6.64 mg PHMB (group 2), respectively. However, the actual weight increase recorded was 23.3 mg PHMB (group 1) and 23.9 mg PHMB (group 2), respectively, which leaves 17.08 mg and 17.27 mg PHMB unaccountable by physical entrapment (see figure 5). Therefore it appears that other factors such as electrostatic interaction may account for the observed strong PHMB absorption to the silk protein membrane 40, yielding a nearly 20 % dry weight increase (19.55 %, group 1 and 18.12 %, group 2) after loading of the silk material with PHMB. Release of PHMB in 0.9 % NaCl was remarkably slower with only 50 % of total PHMB released after 48 hours compared to 90 % of total PHMB released in d H₂O (see also figure 3). Hence, it may be possible that shielding effects of ions influences the electrostatic interaction between the polymeric cationic antimicrobial 55 (PMBH) and the silk protein membrane 40.

### Example 3

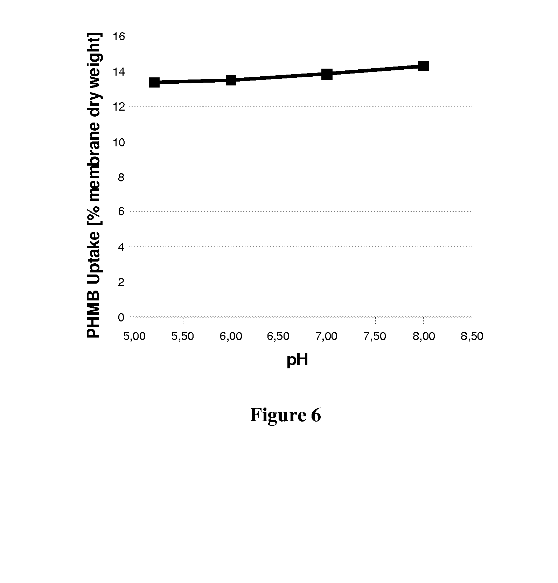

Silk protein membranes 40 were prepared as described in Example 2. Loading was performed for 16 hours at room temperature in four separate groups in 20 ml of 5 % PHMB with pH adjusted to 5.2" 6.0, 7.0 and 8.0. PHMB uptake was then measured as percentage of membrane dry weight. The results are shown in figure 6. The PHMB loading was highest for the silk protein membranes 40 incubated with the polymeric cationic antimicrobial 55 (PMBH) at pH 8.

### Example 4

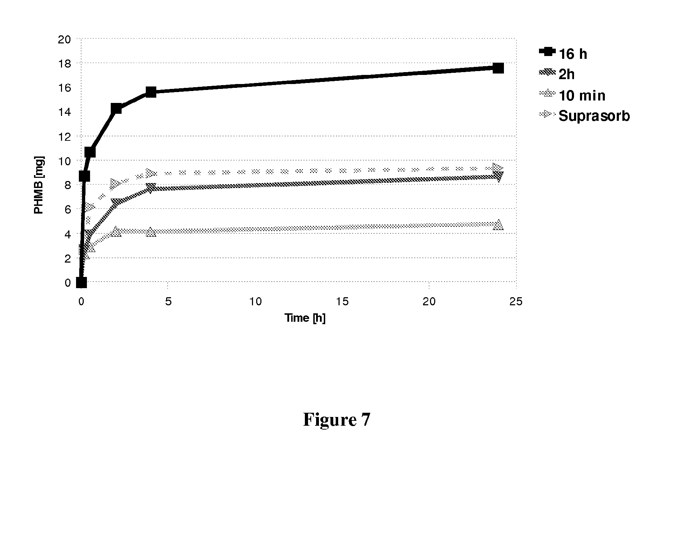

The silk protein membranes 40 were prepared as described in Example 2. The loading was performed for 10 minutes, 2 and 16 hours at 37°C in 20 ml of 5% PHMB. As negative controls, the silk protein membranes 40 were incubated in d H₂O only. PHMB release for up to 24 hours was then measured as described in Example 2. The release profiles shown in figure 7 (negative controls not shown) demonstrate that the incubation time of the silk protein membranes 40 in PHMB solution determines the amount of PHMB released.

### Example 5

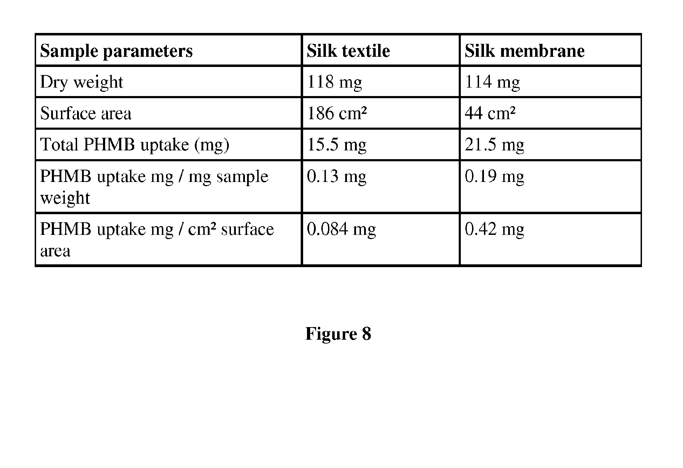

A single layer woven silk textile sample was cut into rectangular shaped samples and weighted. Silk protein membranes 40 were prepared as described in Example 2. The average dry weights of the silk textile samples and the silk protein membranes 40 were comparable with 118 mg (textile) and 114 mg (membrane), respectively (see figure 8). However, both of the sample differed with regard to their surface area with 186 cm² estimated for the woven silk textile sample and 44 cm² for the silk protein membranes 40. The polymeric cationic antimicrobial 55 (PMBH) uptake per g dry weight was comparable for both samples with 0.13 mg PHMB taken up by the woven silk textile sample and 0.19 mg by the silk protein membranes 40. The data suggest that the more than a four-fold increase in surface area of the textile sample does not lead to an increase in the amount of polymeric cationic antimicrobial 55 (PMBH) loaded on the woven silk textiles when compared to the silk protein membranes 40. When considered in relation to sample weight, the uptake of PHMB is roughly comparable for the woven silk textiles and silk protein membranes 40. Hence, loading of the polymeric cationic antimicrobial 55 (PMBH) to the silk protein material appears to be governed by the amount of silk protein material available for loading with polymeric cationic antimicrobial 55 (PMBH); the influence of surface area appears to be of less importance.

### Example 6

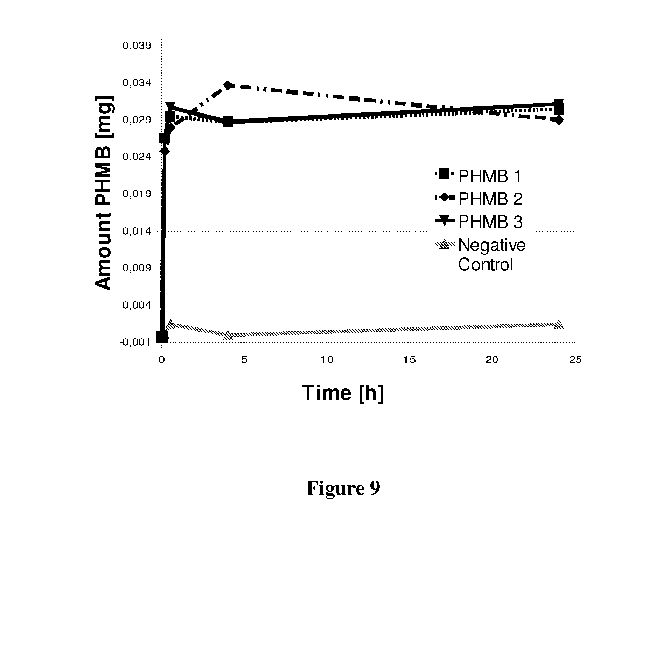

The silk protein fibre 50 was biomimetically spun as described by the applicants in EP 1244828 and WO 2008/052755. Three silk protein fibre 50 samples (length 15 cm) were incubated for 12 hours at room temperature in 3% polymeric cationic antimicrobial 55 (PMBH) solution and dried. The polymeric cationic antimicrobial 55 (PMBH) release profile (see figure 9) was measured, as described in Example 2. The incubated silk protein fibre 50 demonstrated release of the polymeric cationic antimicrobial 55 (PMBH), a control one of the silk protein fibre 50 (i.e. not incubated) showed no release of the polymeric cationic antimicrobial 55 (PMBH).

### Example 7

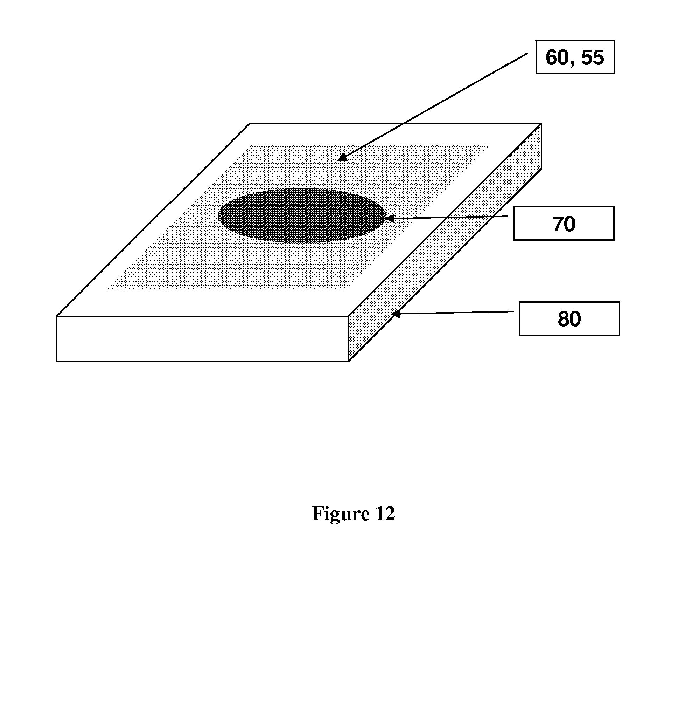

The silk medical device 60 can be used for the treatment of a wound 70. Figure 12 shows the silk medical device 60 placed upon the wound 70 present in skin 80 of a wounded subject. When the silk medical device 60 is placed on the wound 70, the polymeric cationic antimicrobial 55 is released from silk medical device 60 to treat the wound 70.

### Example 8

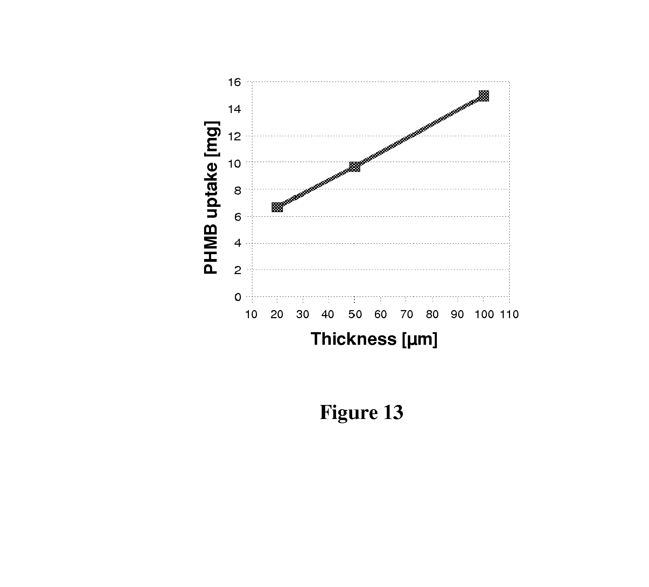

Silk protein membranes 40 were prepared with thicknesses of 20, 50 and 100 µm as described in Example 2. Loading was performed for 24 hours at room temperature in 20 ml of 5 % PHMB. PHMB uptake was then measured by determining the dry weights of each membrane. The results are shown in figure 13. The uptake of PHMB is proportional to the thickness of the membrane and the amount of fibroin, respectively.

### Example 9

Silk protein membranes 40 were prepared as described in Example 2. The loading was performed for 24 hours at room temperature in 20 ml of 5 % PHMB. Antimicrobial activity was demonstrated by a radial diffusion assay on agar inoculated with log-phase E.coli XL1 cells. The silk protein membranes with and without PHMB loaded were placed on top of the agar and incubated at 37°C for 16 hours. The agar around the PHMB membranes exhibited clear zones (halos) confirming antimicrobial activity. The agar around the control membranes showed no antimicrobial activity.

Having thus described the present invention in detail, it is to be understood that the foregoing detailed description of the invention is not intended to limit the scope of the invention thereof. One of ordinary skill in the art would recognise other variants, modifications and alternatives in light of the foregoing discussion.

What is desired to be protected by letters patent is set forth in the following claims.

Reference numerals
- 10: Silk protein solution
- 15: Silk protein material
- 20: Solid support
- 30: Biomimetic Spinning Apparatus
- 40: Silk Protein Membrane
- 50: Silk Protein Fibre
- 55: Polymeric cationic antimicrobial
- 60: Silk Medical Device
- 70: Wound
- 80: Skin

## Claims

1. A method for the manufacture of a medical device (60), the method comprising;
- providing a silk protein material (15, 40, 50), and
- impregnating the silk protein material (40, 50) with a polymeric cationic antimicrobial (55).

2. The method according to claim 1, wherein impregnating the silk protein material (40, 50) with the polymeric cationic antimicrobial (55) involves immersing the silk protein material (40, 50) in a solution of the polymeric cationic antimicrobial (55).

3. The method according to any one of the above claims, wherein the silk protein material (15, 40, 50) is selected from at least one of a silk protein membrane (40) or a silk protein fibre (50).

4. The method according to any one of the above claims, wherein the polymeric cationic antimicrobial (55) is polyhexamethylene biguanide (PHMB).

5. A medical device (60) comprising a silk protein material (15, 40, 50) impregnated with a polymeric cationic antimicrobial (55).

6. The silk medical device (60) according to claim 5, wherein the silk protein material (15, 40, 50) is selected from at least one of a silk protein membrane (40) or a silk protein fibre (50).

7. The silk medical device (60) according to any one of claims 5 to 6, wherein the polymeric cationic antimicrobial (55) is polyhexamethylene biguanide (PHMB).

8. The silk medical device according any one of claims 5 to 7 , wherein the polymeric cationic antimicrobial (55) is impregnated in the silk medical device (60) between 0.1% and 30% dry weight of the silk medical device (60).

9. A kit comprising;
- a silk medical device (60) comprising a silk protein material impregnated (15, 40, 50) with a polymeric cationic antimicrobial (55),
- a container containing said silk medical device (60); and
- instructions for applying the silk medical (60) device to a wound (70).

10. The kit according to claim 9, wherein the kit is sterilised using gamma radiation.
